# EUROPEAN PATENT APPLICATION

(11) **EP 3 284 387 A1**
(43) Date of publication of application: **21.02.2018**
(21) Application number: 16779995.6
(22) Date of filing: 11.04.2016
(51) Int. Cl.: A61B 1/00, G01N 21/17

(54) **OPTICAL PROBE**

(30) Priority: 16.04.2015 JP 2015083897; 06.08.2015 JP 2015155591
(71) Applicant: Sumitomo Electric Industries, Ltd., Osaka-shi, Osaka 541-0041 (JP)
(72) Inventor: SASAKI, Dai, Yokohama-shi Kanagawa 244-8588 (JP); TAMEKUNI, Yoshikyo, Yokohama-shi Kanagawa 244-8588 (JP)
(74) Representative: Hoffmann Eitle
(86) International application number: PCT/JP2016/061648
(87) International publication number: WO 2016/167204

(57) **Abstract**

An optical probe that is easy to manufacture and that can capture a tomographic image with high resolution has a proximal end to be connected to an OCT device and a distal end from which observation light exits, and includes an optical fiber, a Grin lens corresponding to a light collection optical system and a light deflection optical system that are optically connected to the optical fiber at the distal end, a sheath that extends in a first direction, that includes a curved surface portion having a curvature in a section perpendicular to the first direction, that accommodates the optical fiber and the Grin lens in the first direction, and that causes the observation light deflected to exit through the curved surface portion laterally, and a compensation portion that is accommodated in the sheath, that extends in the first direction over a range including a part of the optical fiber and the Grin lens, and that compensates an optical aberration that occurs when the observation light passes through the curved surface portion.

## Description

### Technical Field

The present invention relates to an optical probe used for optical coherence tomography (OCT).

### Background Art

Optical coherence tomography (OCT) has been known as a method of measuring the sectional structure of an object such as an organism. During OCT measurement, an optical probe is inserted near the object, and observation light is emitted from the optical probe. The observation light reflected back from the object is captured by the optical probe.

The optical probe includes a sheath through which the observation light passes, an optical fiber that is disposed in the sheath and that guides the observation light between the proximal end and the distal end of the optical probe, and a light deflection optical system that deflects the observation light to the flank of the optical probe. In this case, the observation light deflected by the light deflection optical system passes through the side wall of the sheath. In the case where the sheath is cylindrical, the side wall does not have a curvature in the longitudinal direction but has a curvature in a direction perpendicular to the longitudinal direction. In this case, there is a problem in that the observation light is subjected to a lensing effect at the location at which the sheath has the curvature, the beam shape of the observation light becomes elliptical, and the resolution of a tomographic image to be captured decreases. In JP2004-223269 (PTL 1), JP2011-519692 (PTL 2), JP2009-523581 (PTL 3), JP2008-514383 (PTL 4), and WO2008/081653 (PTL 5), techniques for providing the light deflection optical system with a lens structure that cancels the lensing effect on the sheath are disclosed to deal with the problem.

### Summary of Invention

### Technical Problem

An object of the present invention is to provide an optical probe that is easy to manufacture and that can capture a tomographic image with high resolution.

### Solution to Problem

An optical probe according to the present invention that has a proximal end to be connected to a measurement unit of an OCT device and a distal end from which observation light exits and that includes an optical fiber, a light collection optical system, a light deflection optical system, a sheath, and a compensation portion is provided to solve the problem. The observation light is transmitted between the proximal end and the distal end through the optical fiber of the optical probe according to the present invention. The light collection optical system is optically connected to the optical fiber at the distal end and collects the observation light that exits from the optical fiber. The light deflection optical system is optically connected to the light collection optical system at the distal end and deflects the observation light that exits from the optical fiber. The sheath extends in a first direction, accommodates the optical fiber, the light collection optical system, and the light deflection optical system in the first direction, includes a curved surface portion having a curvature in a section perpendicular to the first direction, and causes the observation light deflected by the light deflection optical system to pass through the curved surface portion in a second direction intersecting the first direction such that the observation light exits from the distal end. The compensation portion is accommodated in the sheath, extends in the first direction over a range including a part of the optical fiber, the light collection optical system, and the light deflection optical system, and compensates an optical aberration that occurs when the observation light passes through the curved surface portion.

The compensation portion of the optical probe according to the present invention may cover an outer circumference of the light deflection optical system, the light collection optical system, and the part of the optical fiber and may be integrally formed with the light deflection optical system, the light collection optical system, and the optical fiber. In this case, the compensation portion may cover the outer circumference of the light deflection optical system, the light collection optical system, and a glass fiber exposed in a manner in which a coating is removed from the optical fiber and may be integrally formed with the light deflection optical system, the light collection optical system, and the optical fiber. The light collection optical system is preferably a Grin lens having a diameter that is larger than a diameter of the glass fiber and that is less than a diameter of the coating, and an outer diameter of the compensation portion around the outer circumference of the glass fiber is preferably equal to the outer diameter of the compensation portion around the outer circumference of the light collection optical system. When the optical fiber, the light collection optical system, and the light deflection optical system that are integrally formed are viewed from a light deflection optical system side in the first direction, an outer edge of the compensation portion is preferably located inside an outer edge of the coating.

In all the above aspects, a refractive index of the compensation portion is preferably larger than a refractive index of a medium filled between the compensation portion and the sheath, and a curvature of a part of the compensation portion through which the observation light passes is preferably less than a curvature of a part of the compensation portion other than the part through which the observation light passes. In this case, the compensation portion may include a first molded part that extends in the first direction and that includes the part through which the observation light passes, and a second molded part that extends in the first direction, that faces the part through which the observation light passes, and that does not include the part through which the observation light passes, the first molded part and the second molded part may be adjacent to each other across an interface that corresponds to a largest section of the compensation portion, that is perpendicular to a plane extending in the first direction and the second direction, and that is parallel to the first direction, and a center of the optical fiber may be located on a second molded part side from the interface in the section perpendicular to the first direction. In addition, there may be a step on an outer surface of an interface portion between the first molded part and the second molded part.

In all the above aspects, the compensation portion may include, on a part extending in the first direction, a protrusion that protrudes from an outer circumference of the compensation portion toward the sheath.

According to another aspect of the present invention, a method for manufacturing the optical probe according to the present invention is provided. The method includes, defining a space in combination with a first mold for forming a first molded part of the compensation portion that extends in the first direction and that includes a part through which the observation light passes and a second mold for forming a second molded part of the compensation portion that is adjacent to and faces the first molded part and that does not include the part through which the observation light passes, disposing the part of the optical fiber, the light collection optical system, and the light deflection optical system inside the space, and filling the inside of the space with a resin and curing the resin.

In the method according to the present invention for manufacturing the optical probe, the first mold and the second mold may be separated from each other such that a center of the optical fiber is located on a second molded part side from an interface between the first molded part and the second molded part in the section perpendicular to the first direction.

### Advantageous Effects of Invention

According to the present invention, an optical probe that is easy to manufacture and that can capture a tomographic image with high resolution can be provided.

### Brief Description of Drawings

Figure 1 is a schematic view of an OCT device including an optical probe according to an embodiment of the present invention.
Figure 2 at a (a) region illustrates the YZ section of the distal end of the optical probe in Fig. 1. Figure 2 at a (b) region illustrates the front of a glass fiber, a coating, a molded portion, and a Grin lens viewed from the distal end of the optical probe in Fig. 1 in the Z-direction.
Figure 3 is a perspective view of the YZ section of the distal end of the optical probe in Fig. 1.
Figure 4 schematically illustrates the functions of the optical probe in Fig. 1.
Figure 5 illustrates the YZ section and the XY section of an optical path of observation light according to a comparative example.
Figure 6 illustrates the YZ section and the XY section of an optical path of observation light according to the embodiment of the present invention.
Figure 7 illustrates the XZ section of an optical probe according to a modification to the embodiment of the present invention.
Figure 8 illustrates a method for manufacturing the optical probe according to the embodiment of the present invention and illustrates the front of an optical fiber and the Grin lens that are disposed inside molds and viewed from an end side of the optical fiber.
Figure 9 illustrates the method according to another embodiment of the present invention for manufacturing the optical probe and illustrates the front of the optical fiber and the Grin lens that are disposed inside molds and viewed from an end side of the optical fiber.
Figure 10 illustrates the method for manufacturing an optical probe according to a modification to the embodiment of the present invention and illustrates the front of the optical fiber and the Grin lens that are disposed inside molds and viewed from an end side of the optical fiber.

### Description of Embodiments

A specific example of an optical probe according to an embodiment of the present invention will hereinafter be described with reference to the drawings. It is intended that the present invention is not limited to the embodiment, is shown by the scope of claims, and includes all modifications having the same content and range as the scope of claims. In the following description of the drawings, identical components are designated by the same reference numbers, and a duplicated description is omitted.

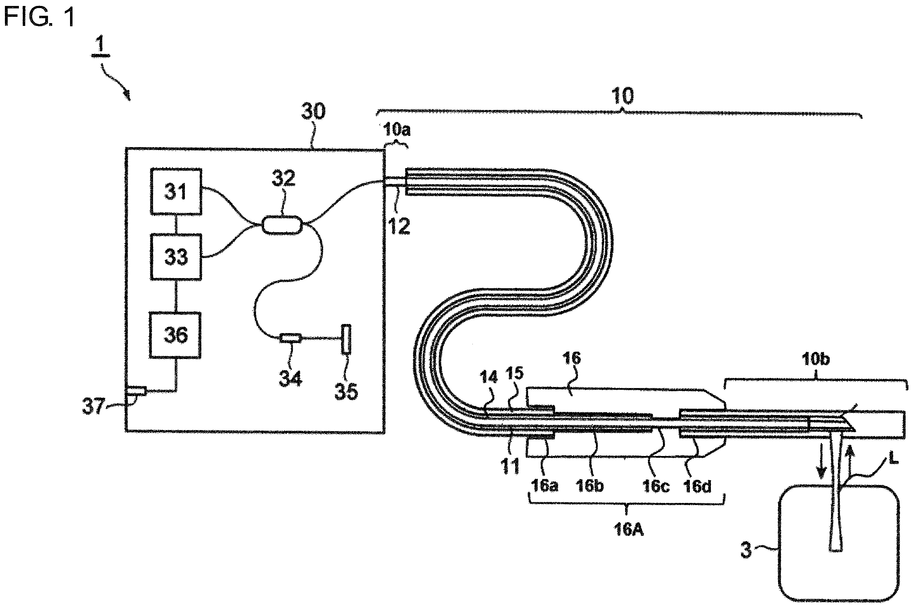

Figure 1 is a schematic view of an OCT device 1 including an optical probe 10 according to the embodiment of the present invention. The OCT device 1 includes the optical probe 10 and a measurement unit 30 and captures an optical coherence tomography image of an object 3. The optical probe 10 has a proximal end 10a and a distal end 10b and includes a handpiece 16 therebetween. An optical fiber 11 extends from the proximal end 10a to the distal end 10b and is inserted through a through-hole 16A of the handpiece 16. The distal end 10b of the optical probe 10 can be inserted into an organism that is an object to be observed while the handpiece 16 is held such that the tip of the distal end 10b is located near a portion of the object to be observed.

The measurement unit 30 includes a light source 31, a bifurcated portion 32, a detector 33, a terminal 34, a reflecting mirror 35, an analyzer 36, and an output port 37. Light emitted from the light source 31 is bifurcated into observation light and reference light at the bifurcated portion 32. The observation light reaches the proximal end 10a of the optical probe 10, propagates through the optical fiber 11, and is emitted from the distal end 10b to the object 3.

Back-reflection light resulted from the radiation of the observation light to the object 3 is incident again on the optical fiber 11 from the distal end 10b and enters the bifurcated portion 32 from the proximal end 10a. The reference light exits from the terminal 34 toward the reflecting mirror 35, is incident again on the terminal 34, and enters the bifurcated portion 32. The observation light and the reference light incident on the bifurcated portion 32 are combined and interfere with each other at the bifurcated portion 32, and interference light is detected by the detector 33. The spectrum of the interference light is analyzed by the analyzer 36, the distribution of back-reflection efficiency at points on the internal cross-section of the object 3 is calculated. A tomographic image of the object 3 is calculated on the basis of the calculation result, and an image signal is outputted from the output port 37.

Strictly speaking, mechanisms that cause the observation light to return again to the distal end 10b via the object 3 include reflection, refraction, and scattering. However, a difference between these is not essential for the present invention. Accordingly, in the description, a general term of these is referred to as back reflection for simplification.

The optical fiber 11 includes an optical connector 12 near the proximal end 10a and is optically connected to the measurement unit 30 with the optical connector 12 interposed therebetween. The OCT device 1 rotates the optical connector 12 to rotate the optical fiber 11 and scans the observation light in the circumferential direction to capture the optical coherence tomography image of the object 3 within a predetermined range.

The optical probe 10 includes a support tube 14 that covers the outer circumference of the optical fiber 11 and a jacket tube 15 that covers the outer circumference of the support tube 14 nearer than the handpiece 16 to the proximal end 10a. The optical fiber 11 and the support tube 14 are secured to the optical connector 12 and are rotatable with respect to the jacket tube 15.

The support tube 14 is a metallic hollow member and may be a thin tubular pipe member or may be formed in a tube whose flexibility is adjusted in a manner in which metallic fibers are twisted. The inner diameter of the support tube 14 is, for example, 0.4 to 0.6 mm, and a single mode optical fiber a coating of which has an outer diameter of 0.25 mm and that is coated can be inserted through the support tube 14. The thickness of the support tube 14 is preferably about 0.3 mm to 0.7 mm so that the rotational torque of the optical connector 12 can be efficiently transmitted to the distal end 10b. Accordingly, the outer diameter of the support tube is about 1 to 2 mm.

The handpiece 16 has the through-hole 16A through which the optical fiber 11 is inserted. The through-hole 16A is divided into a first section 16a, a second section 16b, a third section 16c, and a fourth section 16d in this order in the direction from the proximal end 10a to the distal end 10b. The first section 16a is a section to which the jacket tube 15 is secured. The second section 16b rotatably accommodates the optical fiber 11 and the support tube 14. The third section 16c rotatably accommodates the optical fiber 11. The fourth section 16d secures the distal end 10b (a metallic tube 17 and a sheath 18 described later) and rotatably accommodates the optical fiber 11.

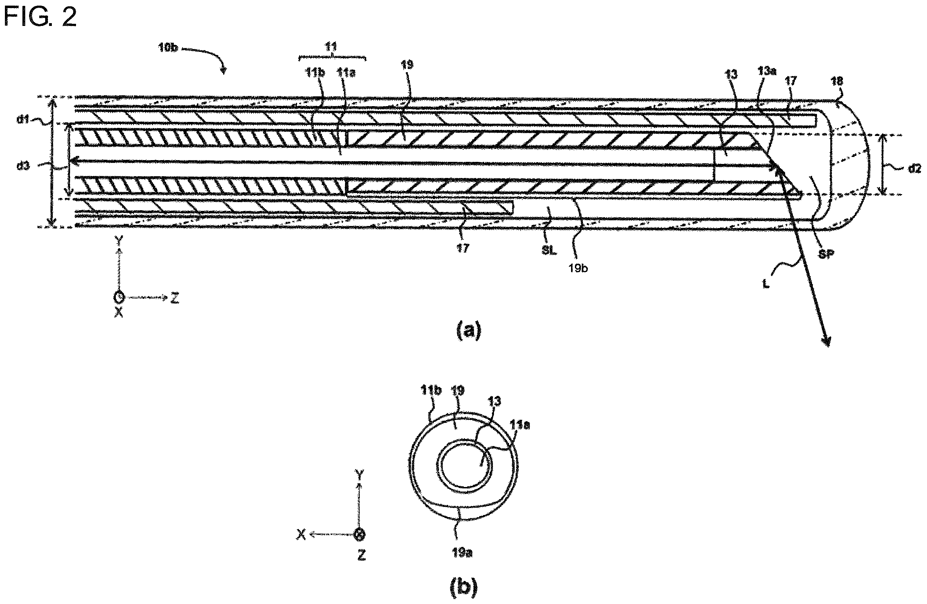

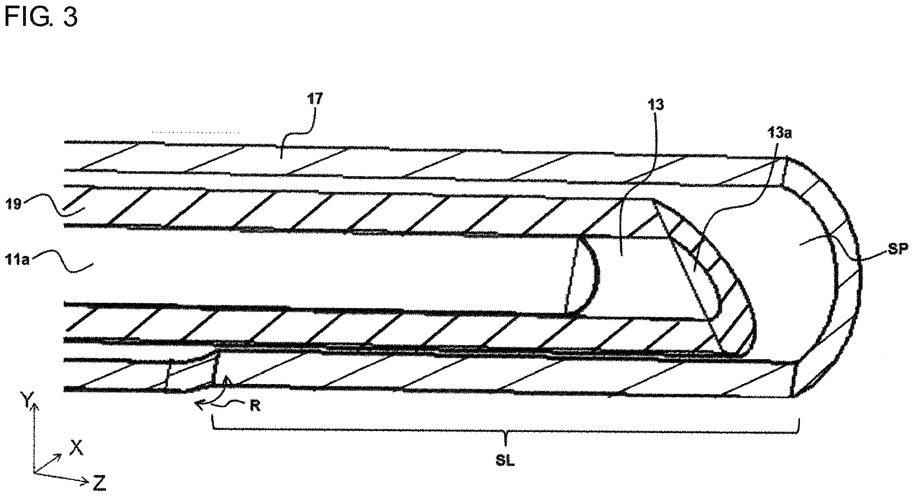

Figure 2 at a (a) region illustrates the YZ section of the distal end 10b of the optical probe 10. Figure 3 is a perspective view of the YZ section of the distal end 10b of the optical probe 10. Figure 2 at the (a) region, Fig. 2 at a (b) region, and Fig. 3 illustrate an XYZ rectangular coordinate system where a direction in which the optical fiber extends coincides with the Z-direction.

As illustrated in Fig. 2 at the (a) region, the distal end 10b includes the optical fiber 11, a Grin lens 13 optically connected to the optical fiber 11, the metallic tube 17 that covers the optical fiber 11 and the Grin lens 13, and the sheath 18 that is made of a resin and that covers the metallic tube 17. The optical fiber 11 and the Grin lens 13 are integrated by a molded portion 19. The molded portion 19 includes a compensation portion 19b at the lower surface from which observation light L exits. The structure and functions of the compensation portion 19b will be described later.

The sheath 18 seals an interior space SP in an airtight manner. The interior space SP may be empty or may be filled with a fluid. The outer diameter d1 of the sheath 18 is 1 mm or less and is preferably less than the outer diameter of the support tube 14. The metallic tube 17 has a slit SL formed in a manner in which a notch extending in the Z-direction from an end portion is made.

The optical fiber 11 is a single mode optical fiber and includes a glass fiber 11, which is formed of a high-refractive-index core (not illustrated) through which light propagates and a low-refractive-index clad (not illustrated) that encompasses the core, and a coating 11b that covers the glass fiber 11a. At the end portion of the optical fiber 11 near the distal end 10b, a part of the coating 11b that has a predetermined length is removed, and the glass fiber 11a is exposed. The Grin lens 13 is connected to the end thereof by fusion bonding. The glass fiber 11a and the Grin lens 13 are encompassed by the molded portion 19. The optical fiber 11, the Grin lens 13, and the molded portion 19 are integrally formed.

The diameters of the glass fiber 11a and the Grin lens 13 in the XY section perpendicular to an optical axis may be equal, or the diameter of the Grin lens may be slightly larger than the diameter of the glass fiber 11a (about 1.02 to 1.10 times the diameter of the glass fiber 11a). A difference in the diameter enables the interface between the Grin lens 13 and the glass fiber 11a to be readily recognized and enables the length of the Grin lens 13 to be readily managed.

The molded portion 19 is formed in a manner in which the glass fiber 11a and the Grin lens 13 are fusion bonded, the optical fiber 11 is subsequently disposed inside molds, and a resin is filled therein and left to cure. The outer diameter of a portion of the molded portion 19 around the outer circumference of the glass fiber 11 a is preferably equal to the outer diameter of a portion of the molded portion 19 around the outer circumference of the Grin lens 13. Thus, the molded portion 19 fills differences in the outer diameter between the glass fiber 11a and the Grin lens 13. Accordingly, the structure of the optical fiber 11, the Grin lens 13, and the molded portion 19 that are integrally formed is highly symmetric about the axis in the Z-direction. This enables a rotational torque to be efficiently transmitted to the distal end 10b in the case where the optical fiber is rotated about the axis in the Z-direction. The molded portion 19 may be formed of a resin through which the observation light L is transmissible, or the glass fiber 11a and the Grin lens 13 may be inserted through a pipe member, such as a glass capillary, formed of a material through which the observation light L is transmissible and may be secured with an adhesive.

The outer diameter d2 of the molded portion 19 is preferably equal to or less than the diameter of the coating 11b so that the optical fiber 11 can be efficiently rotated inside the metallic tube 17. In the case where the optical fiber is a single mode optical fiber, the diameter of the glass fiber 11 a is about 0.125 mm, the diameter of the coating 11b is about 0.25 mm, and the outer diameter d2 of the molded portion 19 is about 0.125 mm to 0.25 mm. In this case, the inner diameter d3 of the metallic tube 17 is preferably about 0.3 to 0.5 mm. The molded portion 19 is preferably formed of a resin, such as a fluorine resin, having a low coefficient of friction.

Figure 2 at the (b) region illustrates the front of the glass fiber 11 a, the coating 11b, the molded portion 19, and the Grin lens 13 viewed from the distal end of the optical probe 10 in the Z-direction. The Grin lens that corresponds to a light collection optical system and a light deflection optical system is formed so as to be contained inside the section of the coating. The molded portion 19 and the compensation portion 19a, which is a part thereof, are formed so as to be contained inside the section of the coating. Thus, the optical fiber 11, the molded portion 19, and the Grin lens 13 that rotate inside the distal end 10b are formed so as to be tapered as a whole. Accordingly, when a rotational torque is transmitted to the distal end 10b through the optical fiber 11, an end of the optical fiber 11 can be prevented from being displaced from a Z-axis, which is a rotational axis, and prevented from moving violently in the sheath 18, and the rotational torque can be efficiently transmitted to the light deflection optical system.

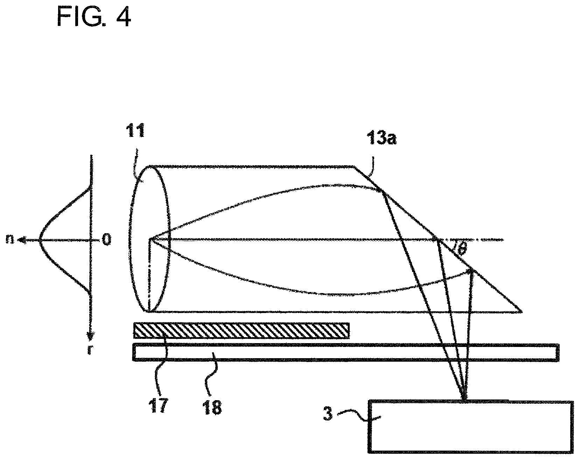

Figure 4 schematically illustrates the functions of the optical probe 10. At the distal end 10b, the edge surface of the Grin lens 13 includes a reflective surface 13a inclined to the Z-axis at an angle θ. A difference between the refractive index of the Grin lens 13 and the refractive index of the interior space SP enables light to be totally reflected and deflected. Accordingly, the Grin lens 13 has a function of serving as the light deflection optical system according to the present invention.

The Grin lens 13 also has a function of serving as the light collection optical system according to the present invention, collects light that has exited from the core of the optical fiber 11, and causes the light to exit therefrom. The Grin lens 13 has a refractive index distribution such that as a distance r from the optical axis extending in the Z-direction increases, the refractive index n gradually decreases, and the refractive index n is expressed as a quadratic function of the distance r. The refractive index of the Grin lens is rotationally symmetric about a central axis. Thus, light that has propagated in the fundamental mode of the optical fiber 11, exited from the core at the edge surface, and diverged converges while propagating substantially parallel to the Z-direction in the inside. During the convergence, the light is deflected by the reflective surface 13a. Thus, the light can be collected near a certain point at the outside.

According to the embodiment, although the Grin lens 13 that has the function of serving as the light collection optical system and the function of serving as the light deflection optical system is used, both the functions may be separated to different members. That is, the Grin lens 13 does not have the reflective surface 13a but has an edge surface perpendicular to the Z-axis, and has only the function of serving as the light collection optical system. A member having the function of serving as the light deflection optical system, such as a prism having the reflective surface 13a, may be secured to the edge surface.

The metallic tube 17 has the slit SL formed in a manner in which the notch extending in the Z-direction from the end portion is made. The sheath 18 and the molded portion 19 are formed of a material through which the observation light L propagating through the optical fiber 11 is transmissible. Thus, the observation light L propagating through the optical fiber 11 is deflected in the Y-direction by the reflective surface 13a while being collected by the Grin lens 13, subsequently passes through the interior space SP, the slit SL, and the sheath 18, and is incident on the object 3 located on the flank of the distal end 10b. The angle θ formed between the reflective surface 13a and the central axis is preferably determined to be no less than 20° and less than 45° such that the observation light L exits slightly oblique to the Z-direction with respect to the Y-direction.

In the optical probe 10, the optical fiber 11 and the support tube 14 can rotate about the axis inside the jacket tube 15 while the optical connector 12 rotates. The rotational torque of the support tube 14 and the optical fiber 11 is transmitted to the optical fiber 11 inside the distal end 10b through the optical fiber 11 held in the through-hole of the handpiece 16. Accordingly, the optical fiber 11 and the Grin lens 13 can be rotated about the Z-axis as the rotational axis inside the metallic tube 17 and the sheath 18 at the distal end 10b in a manner in which the optical connector 12 is rotated.

In the optical probe 10, the optical fiber 11 can rotate about the axis inside the metallic tube 17 having the slit SL. This enables the distal end to be suitably prevented from moving violently from the rotational axis toward the outside due to deformation of the sheath 18 when an external force is applied to the sheath 18 via the handpiece 16.
The observation light that is scanned over the object on the flank of the optical probe 10 is limited by an opening range R about the Z-axis of the slit SL. This inhibits a region of the object 3 other than the region to be observed from being irradiated with the observation light L and prevents an unexpected portion from damaging.

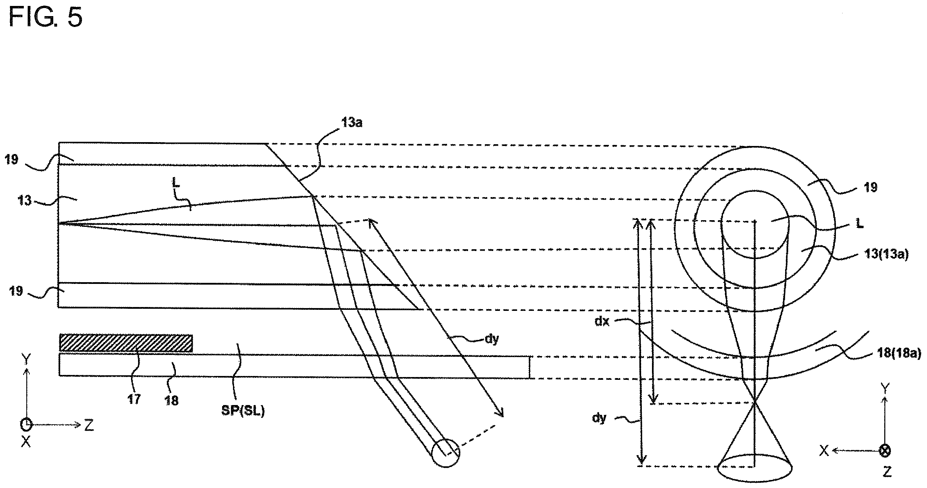

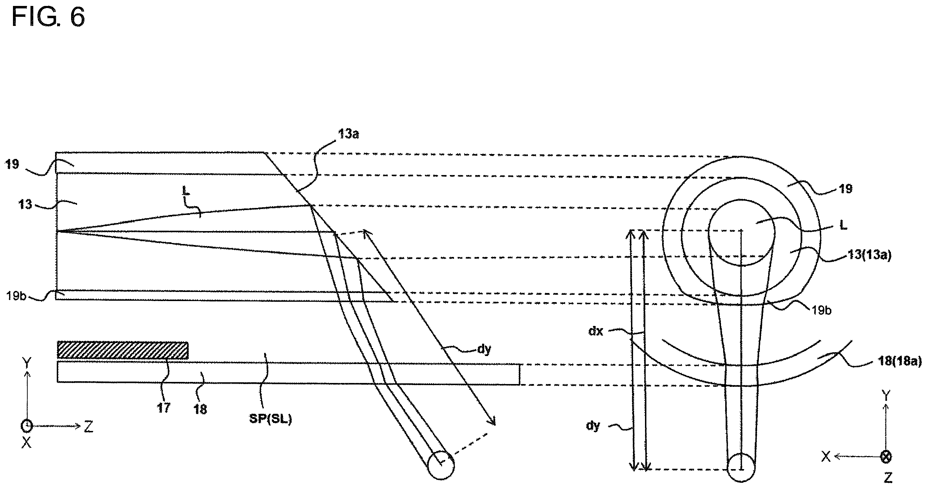

Figure 5 illustrates the YZ section and the XY section of an optical path of the observation light L according to a comparative example. Figure 6 illustrates the YZ section and the XY section of an optical path of the observation light L according to the embodiment. The embodiment differs from the comparative example in that the molded portion 19 includes the compensation portion 19b. The other structures are common. The refractive index of the Grin lens 13 is about 1.45. The refractive index of the molded portion 19 is about 1.45. The refractive index of the interior space SP is about 1.00.
The refractive index of the sheath 18 is about 1.64. The refractive index on the outside of the optical probe 10 is about 1.30. The inclination angle of the reflective surface 13a is about 35°.

As illustrated in the YZ section in Figs. 5 and 6, while the observation light L propagates through the Grin lens 13, the beam diameter thereof increases, and the observation light L is collected and deflected slightly oblique to the Z-direction from the Y-direction by the reflective surface 13a. The observation light L passes through the interior space SP, the slit SL, and the sheath 18 and exits on the flank of the distal end 10b. Since the Grin lens 13 and the molded portion 19 have substantially the same refractive index, the observation light L is scarcely refracted at the interface therebetween. However, the observation light L is refracted at the interface between the molded portion 19 and the interior space SP, which is an interface at which a difference in the refractive index is large, at the interface between the interior space SP and the sheath 18, and at the interface between the sheath 18 and the outside of the optical probe 10 and exits slightly oblique to the Z-direction from the Y-direction.

The optical fiber 11 and the Grin lens are columnar. The molded portion 19 and the sheath 18 are cylindrical. In particular, the sheath 18 extends in the Z-direction and has a curved surface portion 18a having a curvature in the XY section (section perpendicular to a first direction). The observation light L deflected by the reflective surface 13a passes through the curved surface portion 18a so as to exit from the flank of the distal end 10b. In the YZ section, portions at the interface between the molded portion 19 and the interior space SP, at the interface between the interior space SP and the sheath 18, and at the interface between the sheath 18 and the outside of the optical probe 10 do not have a curvature. In the XY section, however, portions at the interface between the molded portion 19 and the interior space SP, at the interface between the interior space SP and the sheath 18 (curved surface portion 18a), and at the interface between the sheath 18 (curved surface portion 18a) and the outside of the optical probe 10 have a curvature. Accordingly, in some cases, a focal length dy in the Y-direction is different from a focal length dx in the X-direction.

According to the comparative example, the observation light L is collected in the XY plane at an interface with an arcuate section between the molded portion 19 and the interior space SP, diverged at an interface with an arcuate section between the interior space SP and the sheath 18, and collected at an interface with an arcuate section between the sheath 18 and the outside of the optical probe 10. At this time, a converging angle in the XY plane is larger than a converging angle in the YZ plane. Accordingly, dy > dx holds, and the beam shape of the observation light L at a focal position in the Y-direction is an ellipse whose longitudinal axis extends in the Z-direction. In the case where the observation light L passes through a passage having an asymmetric refraction power, the observation light L causes an optical aberration, which results in a decrease in the resolution of the tomographic image by the OCT measurement.

According to the embodiment, the molded portion 19 includes the compensation portion 19b. The compensation portion 19b includes a part of the molded portion 19 through which the observation light L passes, and the curvature of the compensation portion 19b is less than the curvature of a portion other than the compensation portion 19b. In this case, the observation light L is moderately collected in the XY plane at the interface between the compensation portion 19b and the interior space SP, diverged at an interface with an arcuate section between the interior space SP and the sheath 18, and collected at an interface with an arcuate section between the sheath 18 and the outside of the optical probe 10. In the case where the curvature of the compensation portion 19b is thus decreased, the focal position of the observation light L is adjusted such that dy = dx holds, and an optical aberration that occurs when the observation light L passes through the curved surface portion 18a of the sheath 18 is compensated. Accordingly, the observation light L with a high degree of circularity can be obtained at the XY focal position. The shape of the compensation portion 19b is not limited to the shape of a spherical lens and may be the shape of a non-spherical lens. The compensation portion 19b may include portions having different curvatures.

As illustrated in Fig. 2 at the (a) region, the compensation portion 19b extends in the Z-direction over a range including the Grin lens 13 and the glass fiber 11a exposed from the optical fiber 11. According to the techniques disclosed in PTL 1 to PTL 5, the light deflection optical system has a lens structure. Accordingly, the techniques have problems in that the process of the light deflection optical system is costly, and positioning thereof against the optical fiber is difficult. However, in the case where the compensation portion is formed as a structure extending over a wide range in the above manner, the compensation portion 19b can be formed on the molded portion 19 by using an easy process such as resin molding or adhesion of a glass capillary.

In particular, according to the embodiment, the molded portion 19 including the compensation portion 19b covers the outer circumference of the Grin lens 13 and the glass fiber 11a to integrally form the optical fiber 11 and the Grin lens 13. This enables the position of the compensation portion 19b to be precisely set with respect to the optical fiber 11 and the Grin lens 13. An increase in the diameter of the molded portion 19 can be prevented in a manner in which the molded portion 19 covers only the glass fiber 11a, and hence, an increase in the diameter of the sheath 18 can be prevented. For example, in the case where the molded portion 19 is formed of a glass capillary, a clearance between the optical fiber 11 or the Grin lens and the glass capillary can be decreased. This makes it easy to arrange the optical fiber 11 and the Grin lens linearly in the Z-axis. Modification

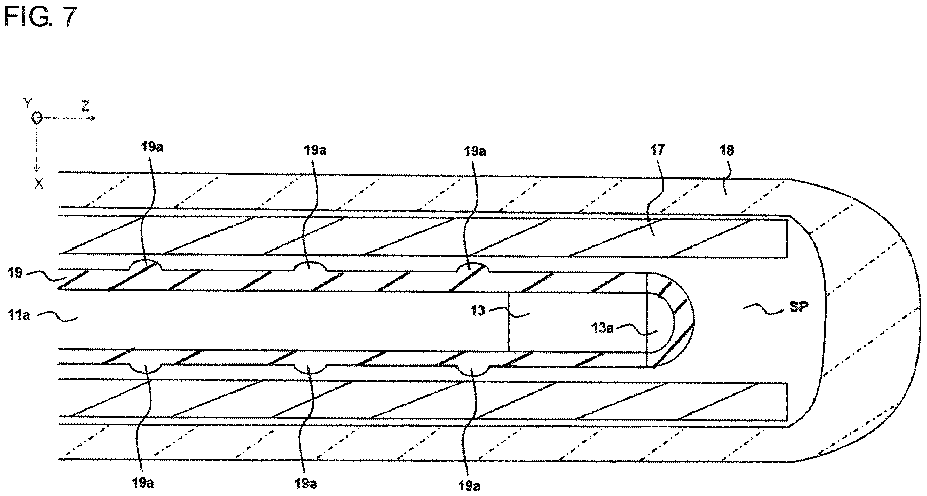

Figure 7 illustrates the XY section of the distal end 10b of an optical probe according to a modification to the embodiment. The modification differs from the above embodiment in that the molded portion 19 includes protrusions 19a. The protrusions 19a are formed on parts of the molded portion 19 extending in the Z-direction so as to protrude from the outer circumference toward the sheath 18. It is preferable that the protrusions 19a be integrally formed with the molded portion 19 by using the same resin when the molded portion 19 is disposed around the outer circumference of the Grin lens 13 and the optical fiber 11.

The protrusions 19a enable the optical fiber 11 to be located at the central position of the sheath 18. That is, in the case where the optical fiber 11 is rotated about the Z-axis as the rotational axis, the protrusions 19a can come in contact with the inner circumference of the sheath 18 (inner circumference of the metallic tube 17) and restrict movement of the optical fiber 11 even when the optical fiber 11 violently moves in the sheath 18. Accordingly, the rotational torque can be efficiently transmitted to the light deflection optical system.

Some of the protrusions 19a are preferably arranged in a section perpendicular to the axis of the molded portion 19. For example, four protrusions are arranged in the circumferential direction at an interval of 90°, or three protrusions are arranged in the circumferential direction at an interval of 120°. Some of the protrusions 19a are preferably arranged in the Z-direction. In this case, the optical fiber 11 and the Grin lens 13 can be readily located at the central position of the cylindrical sheath 18 in a manner in which the heights of the protrusions 19a are equal. It is preferable that the height of each protrusion 19a be determined such that the top thereof is higher than the coating 11b and there is a small clearance between the inner diameter of the mold metallic tube 17 and the protrusion 19a.

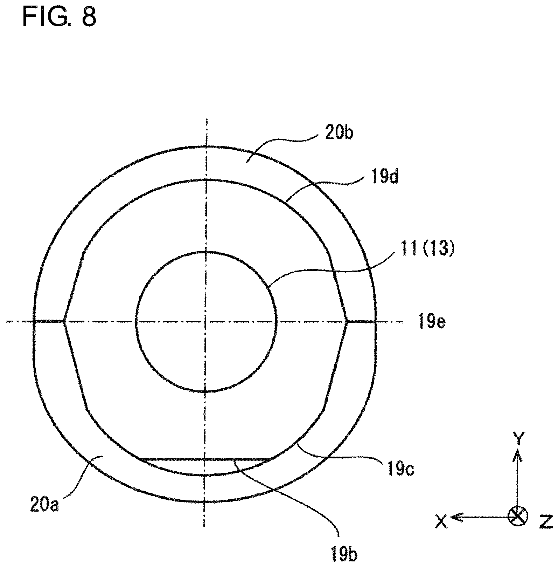

Figure 8 illustrates an example of a method for manufacturing an optical probe according to the present invention and illustrates the front of the optical fiber and the Grin lens that are disposed inside molds and viewed from an end side of the optical fiber 11 (Grin lens 13) in the first direction (Z-direction). The molded portion 19, which covers a part of the optical fiber, the light collection optical system, and the light deflection optical system, includes a first molded part 19c that extends in the first direction and that includes a portion (compensation portion 19b) through which the observation light passes and a second molded part 19d that extends in the first direction, that faces the compensation portion 19b, and that does not include the compensation portion 19b.

A part of the optical fiber 11 and the Grin lens 13 are disposed in a space defined in combination with a first mold (lower mold) 20a for forming the first molded part 19c and a second mold (upper mold) 20b for forming the second molded part 19d, the inside of the space therearound is filled with a resin, and the resin is left to cure. After removal from the molds, the compensation portion integrally formed so as to cover the circumference of the Grin lens 13 and the part of the optical fiber 11 is obtained. The resin is preferably an ultraviolet curable resin but is not limited thereto. According to the example of the method for manufacturing an optical probe, an optical probe that includes the compensation portion having no discontinuity and that enables accurate measurement of the observation light can be obtained.

During the removal from the molds after the resin cures, the end of a portion around a mold opening needs to be wide to facilitate the removal. In the case where the maximum outer diameter is limited as in the optical probe according to the present invention, the size of the compensation portion 19b needs to be decreased accordingly.

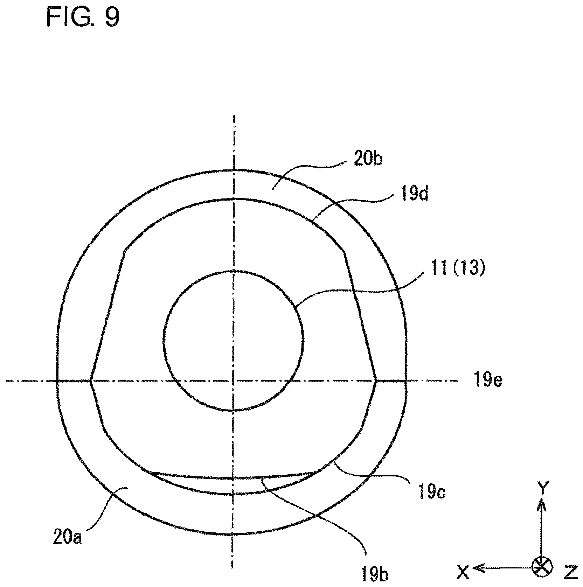

In this case, the size of the compensation portion 19b can be increased in a manner in which a location at which the two molds are separated is changed. As illustrated in Fig. 9, a parting line 19e of the first mold 20a and the second mold 20b is determined such that a line connecting interface portions between the first molded part 19c and the second molded part 19d in the section perpendicular to the first direction (Z-direction) to each other passes nearer than the center of the optical fiber 11 (Grin lens 13) in this section to the side of the first molded part 19c including the compensation portion 19b. This enables tapered portions on the side of the first molded part 19c to be shorter than those on the side of the second molded part 19d. (In this case, the center of the optical fiber is located on the second molded part side from the interface between the first molded part and the second molded part in the section perpendicular to the first direction.) Consequently, the size (corresponding to the width in the X-direction in Fig. 9) of the compensation portion 19b can be increased.

Although, according to the above example, the parting line 19e of the first mold 20a and the second mold 20b is determined so as to be bilaterally symmetric about an axis that passes through the center of the optical fiber 11 (Grin lens 13) and that is parallel to the Y-axis in Fig. 9, the parting line is not limited thereto, provided that the parting line does not cross the compensation portion 19b. The mold for forming the second molded part 19d including no compensation portion 19b does not need to be formed of one body but may be separated to two or more bodies.

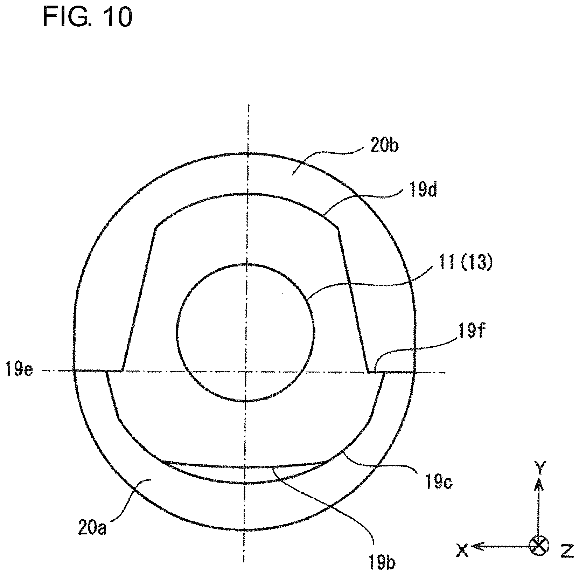

Another modification will be described. Figure 10 illustrates the front of another optical fiber viewed in the first direction (Z-direction). A difference from the example in Fig. 9 is that, on the outer surface of the compensation portion, steps 19f are formed at the interface portions between the first molded part 19c and the second molded part 19d. The step extends in the first direction (Z-direction). An inclined surface (for example, a portion denoted by 13a in Fig. 6) of the end (Grin lens 13) of the optical fiber 11 needs to have a high angular precision with respect to the compensation portion 19b. For this reason, measurement (inspection) for checking whether a desired angle is obtained needs to be performed with high precision. In order to perform the measurement with high precision, it is important for a reference surface or a reference line, which can be a sign during observation from the outside, to be easy to detect. The steps 19f on the outer surface of the interface portions between the first molded part 19c and the second molded part 19d can be readily used for a reference line during the measurement (inspection), and hence, the precision of the measurement (inspection) can be increased.

Each of the steps is preferably 5 µm to 30 µm. In the case where the step is too large, the second molded part 19d becomes too thin. In the case where the step is too small, it is difficult for the step to be used to recognize the reference position.

## Claims

1. An optical probe having a proximal end configured to be connected to a measurement unit of an OCT device and a distal end configured to exit observation light, comprising:
an optical fiber configured to transmit the observation light between the proximal end and the distal end;
a light collection optical system that is optically connected to the optical fiber at the distal end and that is configured to collect the observation light that exits from the optical fiber;
a light deflection optical system that is optically connected to the light collection optical system at the distal end and that is configured to deflect the observation light that exits from the optical fiber;
a sheath that extends in a first direction, that accommodates the optical fiber, the light collection optical system, and the light deflection optical system in the first direction, that includes a curved surface portion having a curvature in a section perpendicular to the first direction, and that in configured to cause the observation light deflected by the light deflection optical system to pass through the curved surface portion in a second direction intersecting the first direction such that the observation light exits from the distal end; and
a compensation portion that is accommodated in the sheath, that extends in the first direction over a range including a part of the optical fiber, the light collection optical system, and the light deflection optical system, and that is configured to compensate an optical aberration that arises from penetration of the observation light through the curved surface portion.

2. The optical probe according to Claim 1,
wherein the compensation portion covers an outer circumference of the light deflection optical system, the light collection optical system, and the part of the optical fiber and is integrally formed with the light deflection optical system, the light collection optical system, and the optical fiber.

3. The optical probe according to Claim 2,
wherein the optical fiber includes, in the sheath, a coating and a glass fiber exposed in a manner in which the coating is removed, and
wherein the compensation portion covers the outer circumference of the light deflection optical system, the light collection optical system, and the glass fiber and is integrally formed with the light deflection optical system, the light collection optical system, and the optical fiber.

4. The optical probe according to Claim 3,
wherein the light collection optical system is a Grin lens having a diameter that is larger than a diameter of the glass fiber and that is less than a diameter of the coating, and
wherein an outer diameter of the compensation portion around the outer circumference of the glass fiber is equal to the outer diameter of the compensation portion around the outer circumference of the light collection optical system.

5. The optical probe according to Claim 3 or Claim 4,
wherein an outer edge of the compensation portion is located inside an outer edge of the coating in a view of the optical fiber, the light collection optical system, and the light deflection optical system that are integrally formed, the view being from a light deflection optical system side in the first direction.

6. The optical probe according to any one of Claims 1 to 5,
wherein a refractive index of the compensation portion is larger than a refractive index of a medium filled between the compensation portion and the sheath, and
wherein a curvature of a part of the compensation portion through which the observation light passes is less than a curvature of a part of the compensation portion other than the part through which the observation light passes.

7. The optical probe according to Claim 6,
wherein the compensation portion includes a first molded part that extends in the first direction and that includes the part through which the observation light passes, and a second molded part that extends in the first direction, that faces the part through which the observation light passes, and that does not include the part through which the observation light passes,
wherein the first molded part and the second molded part are adjacent to each other across an interface that corresponds to a largest section of the compensation portion, that is perpendicular to a plane extending in the first direction and the second direction, and that is parallel to the first direction, and
wherein a center of the optical fiber is located on a second molded part side from the interface in the section perpendicular to the first direction.

8. The optical probe according to Claim 7,
wherein the optical probe has a step on an outer surface of an interface portion between the first molded part and the second molded part.

9. The optical probe according to any one of Claims 1 to 8,
wherein the compensation portion includes, on a part extending in the first direction, a protrusion that protrudes from an outer circumference of the compensation portion toward the sheath.

10. A method for manufacturing the optical probe according to Claim 1, comprising:
defining a space in combination with a first mold for forming a first molded part of the compensation portion that extends in the first direction and that includes a part through which the observation light passes and a second mold for forming a second molded part of the compensation portion that is adjacent to and faces the first molded part and that does not include the part through which the observation light passes;
disposing the part of the optical fiber, the light collection optical system, and the light deflection optical system inside the space; and
filling the inside of the space with a resin and curing the resin.

11. The method according to Claim 10 for manufacturing the optical probe, wherein the first mold and the second mold are separated from each other such that a center of the optical fiber is located on a second molded part side from an interface between the first molded part and the second molded part in the section perpendicular to the first direction.
